# EUROPEAN PATENT SPECIFICATION

(11) **EP 3 369 394 B1**
(45) Date of publication and mention of the grant of the patent: **10.06.2020**
(21) Application number: 18159475.5
(22) Date of filing: 01.03.2018
(51) Int. Cl.: A61B 90/00, A61B 34/20, A61B 34/30, A61B 17/00

(54) **SYSTEM FOR A SURVEILLANCE MARKER IN ROBOTIC-ASSISTED SURGERY**
SYSTEM FÜR EINEN ÜBERWACHUNGSMARKER IN DER ROBOTERGESTÜTZTEN CHIRURGIE
SYSTÈME POUR UN MARQUEUR DE SURVEILLANCE EN CHIRURGIE ASSISTÉE PAR ROBOT

(30) Priority: 03.03.2017 US 201715448670
(43) Date of publication of application: 05.09.2018
(73) Proprietor: Globus Medical, Inc., Audubon, PA 19403 (US)
(72) Inventor: BERKOWITZ, Andrew, PHILADELPHIA, PA 19127 (US); FORSYTH, Jeffrey, CRANSTON, RI 02921 (US); JOSHI, Sanjay M., ANDOVER, MA 01810 (US); CRAWFORD, Neil R., CHANDLER, AZ 85224 (US); JOHNSON, Norbert, NORTH ANDOVER, MA 01845 (US)
(74) Representative: Morabito, Sara

(56) References cited:
- WO-A1-2013/192598
- US-A1- 2009 306 499
- US-A1- 2016 354 153

## Description

### CROSS-REFERENCE TO RELATED APPLICATIONS

This application is a continuation-in-part of U.S. Patent Application No. 15/157,444 filed May 18, 2016, which is a continuation-in-part application of U.S. patent application No. 15/095,883 filed on April 11, 2016 (published as U.S. Patent Publication No. 2016/0220320 A1), which is a continuation-in-part application of U.S. patent application No. 14/062,707 filed on October 24, 2013 (published as U.S. Patent Publication No. 2014/0275955 A1), which is a continuation-in-part application of U.S. patent application No. 13/924,505 filed on June 21, 2013 (published as U.S. Patent Publication No. 2013/0345718 A1, with corrected publication as U.S. Patent Publication No. 2016/0242849 A9), which is a nonprovisional patent application that claims priority to U.S. provisional patent application No. 61/662,702 filed on June 21, 2012, and claims priority to U.S. provisional patent application No. 61/800,527 filed on March 15, 2013.

### FIELD OF THE INVENTION

The present invention relates to surveillance marker implementation for robot-assisted surgical techniques.

### BACKGROUND OF THE INVENTION

Various medical procedures require the accurate localization of a three-dimensional position of a surgical instrument within the body in order to effect optimized treatment. For example, some surgical procedures to fuse vertebrae require that a surgeon drill multiple holes into the bone structure at specific locations. To achieve high levels of mechanical integrity in the fusing system, and to balance the forces created in the bone structure, it is necessary that the holes are drilled at the correct location. Vertebrae, like most bone structures, have complex shapes including non-planar curved surfaces making accurate and perpendicular drilling difficult.

Conventionally, using currently-available systems and methods, a surgeon manually holds and positions a drill guide tube by using a guidance system to overlay the drill tube's position onto a three dimensional image of the anatomical structures of a patient, for example, bone structures of the patient. This manual process is both tedious, time consuming, and error-prone. Further, whether the surgery can be considered successful largely depends upon the dexterity of the surgeon who performs it. Thus, there is a need for the use of robot assisted surgery to more accurately position surgical instruments and more accurately depict the position of those instruments in relation to the anatomical structures of the patient.

Currently, limited robotic assistance for surgical procedures is available. For example, certain systems allow a user to control a robotic actuator. These systems convert a surgeon's gross movements into micro-movements of the robotic actuator to more accurately position and steady the surgical instruments when undergoing surgery. Although these systems may aid in eliminating hand tremor and provide the surgeon with improved ability to work through a small opening, like many of the robots commercially available today, these systems are expensive, obtrusive, and require a cumbersome setup for the robot in relation to the patient and the user (e.g., a surgeon).

In some robotic-assisted systems, registration techniques may be used in order to properly track surgical instruments in relation to 2D and/or 3D images of the patient's target anatomy. As previously discussed in parent applications to the present disclosure (listed above), a dynamic reference base (DRB) may be physically attached to bony structures of a patient. After registration of the markers to images of the patient's anatomy, the DRB may be used as a reference point in order to properly display the position of navigated surgical instruments in relation to images of the patient's anatomy. In the event the DRB is shifted or dislodged, the registration process may need to be reinitiated so ensure proper registration and that the visual display of the navigated instruments relative to images of the patient's anatomy are accurate to real-life movements of the instruments.

One way to determine if the DRB has been dislodged or moved is through the use of a surveillance marker. The use of surveillance makers has been previously described in U.S. Patent Application No. 13/294.505. The surveillance marker is a single tracked marker attached to the patient in a location other than the location of the DRB that tracks patient position. If the patient is moved relative to the tracking cameras, the surveillance marker and DRB would be expected to move together by the same amount, with no relative movement between DRB and surveillance marker. However, movement of the surveillance marker relative to tracking markers on the DRB is an indicator that the DRB may have been accidentally dislodged.

The surveillance marker may require additional preparation and surgical incision of the patient at the location where the surveillance marker is to be applied. Thus, there is a need to allow a surgeon to attach the surveillance marker to the patient using the same incision as was used for DRB placement. To improve functionality, the surveillance marker may not rigidly interface with the DRB.

Accordingly, there exists a need for a surveillance marker that does not rigidly interface with the DRB while still effectively detecting DRB dislodgment. This may be accomplished by the present disclosure by attaching the surveillance marker to bone near the DRB. For example, having the surveillance marker on a post that does not touch the DRB.

WO 2013/192598 A1 discloses a medical robot system, including a robot coupled to an effectuator element with the robot configured for controlled movement and positioning. The system may include a transmitter configured to emit one or more signals, and the transmitter is coupled to an instrument coupled to the effectuator element.

US 2009/306499 A1 discloses computer-based surgical apparatuses for a self-detecting kinematic assembly. The self-detecting kinematic clamp assembly detects and indicates a degradation in pose. The self-detecting kinematic clamp assembly includes a tracking element for tracking a pose of the kinematic clamp assembly.

### SUMMARY OF THE INVENTION

To meet this and other needs, systems for detecting the presence of unintended movement of a surgical instrument during a surgical procedure are provided. The present invention is defined by the features of the independent claim. Preferred embodiments are given in the dependent claims.

### BRIEF DESCRIPTION OF THE DRAWINGS

The invention and the following detailed description of certain embodiments thereof may be understood by reference to the following figures:
FIG. 1 is an overhead view of a potential arrangement for locations of the robotic system, patient, surgeon, and other medical personnel during a surgical procedure;
FIG. 2 illustrates the robotic system including positioning of the surgical robot and the camera relative to the patient according to one embodiment;
FIG. 3 illustrates a surgical robotic system in accordance with an exemplary embodiment;
FIG. 4 illustrates a portion of a surgical robot in accordance with an exemplary embodiment;
FIG. 5 illustrates a block diagram of a surgical robot in accordance with an exemplary embodiment;
FIG. 6 illustrates a surgical robot in accordance with an exemplary embodiment;
FIGS. 7A-7C illustrate an end effector in accordance with an exemplary embodiment;
FIG. 8 illustrates a surgical instrument and the end effector, before and after, inserting the surgical instrument into the guide tube of the end effector according to one embodiment;
FIGS. 9A-9C illustrate portions of an end effector and robot arm in accordance with an exemplary embodiment;
FIG. 10 illustrates a dynamic reference array, an imaging array, and other components in accordance with an exemplary embodiment;
FIG. 11 illustrates a method of registration not forming part of the claimed invention;
FIG. 12A-12B illustrate embodiments of imaging devices according to exemplary embodiments;
FIGs. 13A-13B illustrate a surveillance marker in accordance with exemplary embodiments of the present invention;
FIGs. 14A-14B illustrate a surveillance marker not forming part of the claimed invention;
FIG. 15 illustrates a surveillance marker not forming part of the claimed invention;

### DETAILED DESCRIPTION OF THE INVENTION

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings.

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

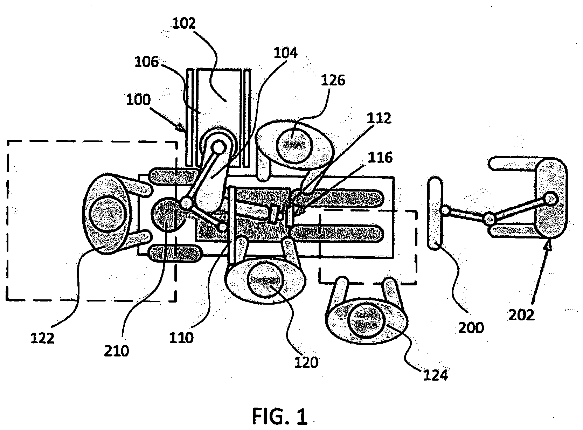

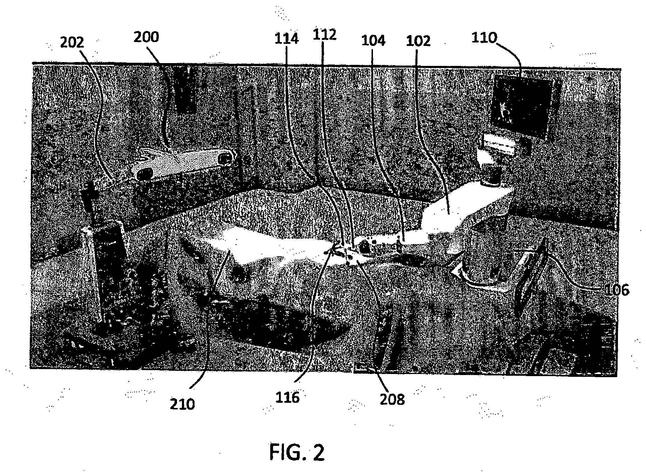

Turning now to the drawing, FIGS. 1 and 2 illustrate a surgical robot system 100 in accordance with an exemplary embodiment. Surgical robot system 100 may include, for example, a surgical robot 102, one or more robot arms 104, a base 106, a display 110, an end effector 112, for example, including a guide tube 114, and one or more tracking markers The surgical robot system 100 includes a patient tracking device 116 also including one or more tracking markers which is adapted to be secured directly to the patient 210 (e.g., to the bone of the patient 210). The surgical robot system 100 utilizes a camera 200, for example, positioned on a camera stand 202. The camera stand 202 can have any suitable configuration to move, orient, and support the camera 200 in a desired position. The camera 200 may include any suitable camera or cameras, such as one or more infrared cameras (e.g., bifocal or stereophotogrammetric cameras), able to identify, for example, active and passive tracking markers in a given measurement volume viewable from the perspective of the camera 200. The camera 200 may scan the given measurement volume and detect the light that comes from the markers in order to identify and determine the position of the markers in three dimensions. For example, active markers may include infrared-emitting markers that are activated by an electrical signal (e.g., infrared light emitting diodes (LEDs)), and passive markers may include retro-reflective markers that reflect infrared light (e.g., they reflect incoming IR radiation into the direction of the incoming light), for example, emitted by illuminators on the camera 200 or other suitable device.

FIGS. 1 and 2 illustrate a potential configuration for the placement of the surgical robot system 100 in an operating room environment. For example, the robot 102 may be positioned near or next to patient 210. Although depicted near the head of the patient 210, it will be appreciated that the robot 102 can be positioned at any suitable location near the patient 210 depending on the area of the patient 210 undergoing the operation. The camera 200 may be separated from the robot system 100 and positioned at the foot of patient 210. This location allows the camera 200 to have a direct visual line of sight to the surgical field 208. Again, it is contemplated that the camera 200 may be located at any suitable position having line of sight to the surgical field 208. In the configuration shown, the surgeon 120 may be positioned across from the robot 102, but is still able to manipulate the end effector 112 and the display 110. A surgical assistant 126 may be positioned across from the surgeon 120 again with access to both the end effector 112 and the display 110. If desired, the locations of the surgeon 120 and the assistant 126 may be reversed. The traditional areas for the anesthesiologist 122 and the nurse or scrub tech 124 remain unimpeded by the locations of the robot 102 and camera 200.

With respect to the other components of the robot 102, the display 110 can be attached to the surgical robot 102 and in other exemplary embodiments, display 110 can be detached from surgical robot 102, either within a surgical room with the surgical robot 102, or in a remote location. End effector 112 may be coupled to the robot arm 104 and controlled by at least one motor. In exemplary embodiments, end effector 112 can comprise a guide tube 114, which is able to receive and orient a surgical instrument 608 (described further herein) used to perform surgery on the patient 210. As used herein, the term "end effector" is used interchangeably with the terms "end-effectuator" and "effectuator element." Although generally shown with a guide tube 114, it will be appreciated that the end effector 112 may be replaced with any suitable instrumentation suitable for use in surgery. In some embodiments, end effector 112 can comprise any known structure for effecting the movement of the surgical instrument 608 in a desired manner.

The surgical robot 102 is able to control the translation and orientation of the end effector 112. The robot 102 is able to move end effector 112 along x-, y-, and z-axes, for example. The end effector 112 can be configured for selective rotation about one or more of the x-, y-, and z- axis, and a Z Frame axis (such that one or more of the Euler Angles (e.g., roll, pitch, and/or yaw) associated with end effector 112 can be selectively controlled). In some exemplary embodiments, selective control of the translation and orientation of end effector 112 can permit performance of medical procedures with significantly improved accuracy compared to conventional robots that utilize, for example, a six degree of freedom robot arm comprising only rotational axes. For example, the surgical robot system 100 may be used to operate on patient 210, and robot arm 104 can be positioned above the body of patient 210, with end effector 112 selectively angled relative to the z-axis toward the body of patient 210.

In some exemplary embodiments, the position of the surgical instrument 608 can be dynamically updated so that surgical robot 102 can be aware of the location of the surgical instrument 608 at all times during the procedure. Consequently, in some exemplary embodiments, surgical robot 102 can move the surgical instrument 608 to the desired position quickly without any further assistance from a physician (unless the physician so desires). In some further embodiments, surgical robot 102 can be configured to correct the path of the surgical instrument 608 if the surgical instrument 608 strays from the selected, preplanned trajectory. In some exemplary embodiments, surgical robot 102 can be configured to permit stoppage, modification, and/or manual control of the movement of end effector 112 and/or the surgical instrument 608. Thus, in use, in exemplary embodiments, a physician or other user can operate the system 100, and has the option to stop, modify, or manually control the autonomous movement of end effector 112 and/or the surgical instrument 608. Further details of surgical robot system 100 including the control and movement of a surgical instrument 608 by surgical robot 102 can be found in co-pending U.S. patent application Ser. No. 13/924,505.

The robotic surgical system 100 can comprise one or more tracking markers configured to track the movement of robot arm 104, end effector 112, patient 210, and/or the surgical instrument 608 in three dimensions. In exemplary embodiments, a plurality of tracking markers can be mounted (or otherwise secured) thereon to an outer surface of the robot 102, such as, for example and without limitation, on base 106 of robot 102, on robot arm 104, or on the end effector 112. In exemplary embodiments, at least one tracking marker of the plurality of tracking markers can be mounted or otherwise secured to the end effector 112. One or more tracking markers can further be mounted (or otherwise secured) to the patient 210. In exemplary embodiments, the plurality of tracking markers can be positioned on the patient 210 spaced apart from the surgical field 208 to reduce the likelihood of being obscured by the surgeon, surgical tools, or other parts of the robot 102. Further, one or more tracking markers can be further mounted (or otherwise secured) to the surgical tools 608 (e.g., a screw driver, dilator, implant inserter, or the like). Thus, the tracking markers enable each of the marked objects (e.g., the end effector 112, the patient 210, and the surgical tools 608) to be tracked by the robot 102. In exemplary embodiments, system 100 can use tracking information collected from each of the marked objects to calculate the orientation and location, for example, of the end effector 112, the surgical instrument 608 (e.g., positioned in the tube 114 of the end effector 112), and the relative position of the patient 210.

In exemplary embodiments, one or more of markers may be optical markers. In some embodiments, the positioning of one or more tracking markers on end effector 112 can maximize the accuracy of the positional measurements by serving to check or verify the position of end effector 112. Further details of surgical robot system 100 including the control, movement and tracking of surgical robot 102 and of a surgical instrument 608 can be found in co-pending U.S. patent application Ser. No. 13/924,505.

Exemplary embodiments include one or more markers coupled to the surgical instrument 608. In exemplary embodiments, these markers, for example, coupled to the patient 210 and surgical instruments 608, as well as markers coupled to the end effector 112 of the robot 102 can comprise conventional infrared light-emitting diodes (LEDs) or an Optotrak® diode capable of being tracked using a commercially available infrared optical tracking system such as Optotrak®. Optotrak® is a registered trademark of Northern Digital Inc., Waterloo, Ontario, Canada. In other embodiments, markers can comprise conventional reflective spheres capable of being tracked using a commercially available optical tracking system such as Polaris Spectra. Polaris Spectra is also a registered trademark of Northern Digital, Inc. In an exemplary embodiment, the markers coupled to the end effector 112 are active markers which comprise infrared light-emitting diodes which may be turned on and off, and the markers coupled to the patient 210 and the surgical instruments 608 comprise passive reflective spheres.

In exemplary embodiments, light emitted from and/or reflected by markers can be detected by camera 200 and can be used to monitor the location and movement of the marked objects. In alternative embodiments, markers can comprise a radio-frequency and/or electromagnetic reflector or transceiver and the camera 200 can include or be replaced by a radio-frequency and/or electromagnetic transceiver.

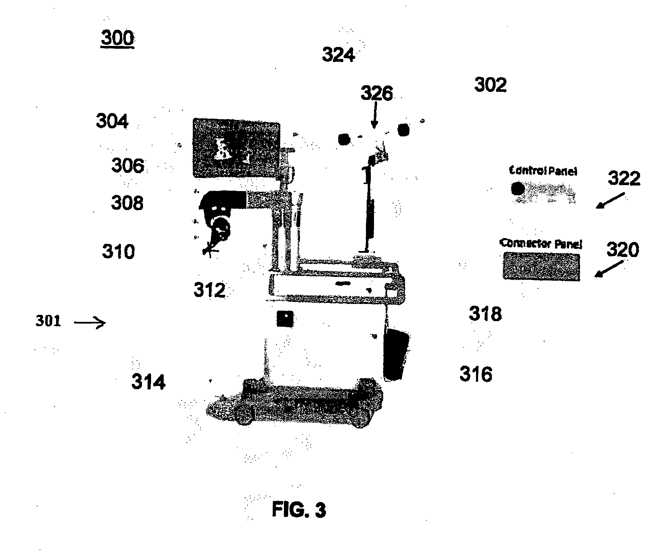

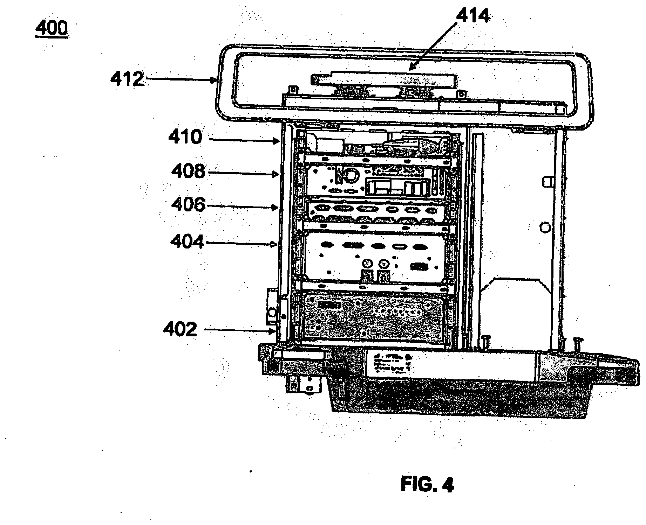

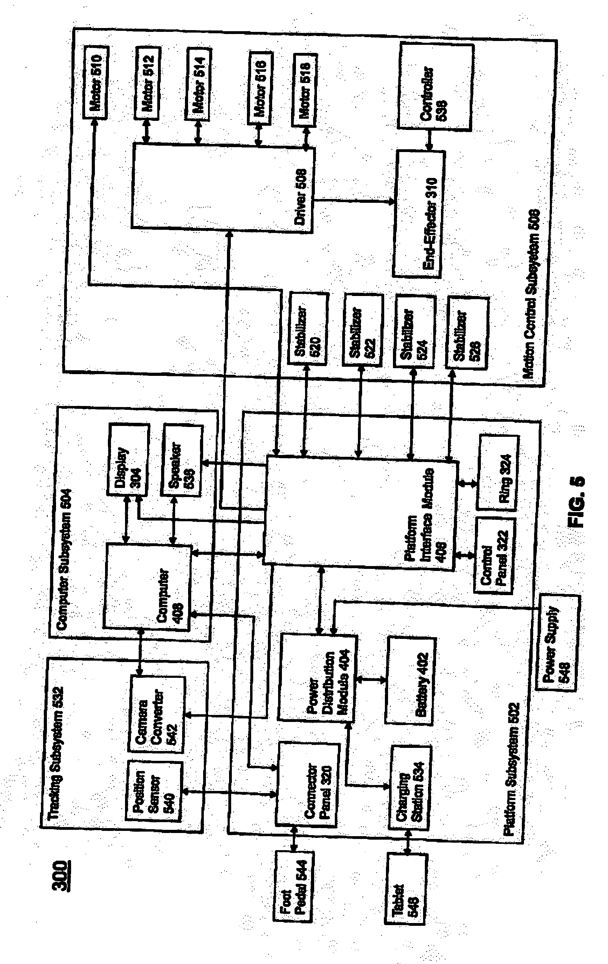

Similar to surgical robot system 100, FIG. 3 illustrates a surgical robot system 300 and camera stand 302, in a docked configuration, consistent with an exemplary embodiment of the present disclosure. Surgical robot system 300 may comprise a robot 301 including a display 304, upper arm 306, lower arm 308, end effector 310, vertical column 312, casters 314, cabinet 316, tablet drawer 318, connector panel 320, control panel 322, and ring of information 324. Camera stand 302 may comprise camera 326. These components are described in greater with respect to FIG. 5. FIG. 3 illustrates the surgical robot system 300 in a docked configuration where the camera stand 302 is nested with the robot 301, for example, when not in use. It will be appreciated by those skilled in the art that the camera 326 and robot 301 may be separated from one another and positioned at any appropriate location during the surgical procedure, for example, as shown in FIGS. 1 and 2. FIG. 4 illustrates a base 400 consistent with an exemplary embodiment of the present disclosure. Base 400 may be a portion of surgical robot system 300 and comprise cabinet 316. Cabinet 316 may house certain components of surgical robot system 300 including but not limited to a battery 402, a power distribution module 404, a platform interface board module 406, a computer 408, a handle 412, and a tablet drawer 414. The connections and relationship between these components is described in greater detail with respect to FIG. 5.

FIG. 5 illustrates a block diagram of certain components of an exemplary embodiment of surgical robot system 300. Surgical robot system 300 may comprise platform subsystem 502, computer subsystem 504, motion control subsystem 506, and tracking subsystem 532. Platform subsystem 502 may further comprise battery 402, power distribution module 404, platform interface board module 406, and tablet charging station 534. Computer subsystem 504 may further comprise computer 408, display 304, and speaker 536. Motion control subsystem 506 may further comprise driver circuit 508, motors 510, 512, 514, 516, 518, stabilizers 520, 522, 524, 526, end effector 310, and controller 538. Tracking subsystem 532 may further comprise position sensor 540 and camera converter 542. System 300 may also comprise a foot pedal 544 and tablet 546.

Input power is supplied to system 300 via a power source 548 which may be provided to power distribution module 404. Power distribution module 404 receives input power and is configured to generate different power supply voltages that are provided to other modules, components, and subsystems of system 300. Power distribution module 404 may be configured to provide different voltage supplies to platform interface module 406, which may be provided to other components such as computer 408, display 304, speaker 536, driver 508 to, for example, power motors 512, 514, 516, 518 and end effector 310, motor 510, ring 324, camera converter 542, and other components for system 300 for example, fans for cooling the electrical components within cabinet 316.

Power distribution module 404 may also provide power to other components such as tablet charging station 534 that may be located within tablet drawer 318. Tablet charging station 534 may be in wireless or wired communication with tablet 546 for charging table 546. Tablet 546 may be used by a surgeon consistent with the present disclosure and described herein. Power distribution module 404 may also be connected to battery 402, which serves as temporary power source in the event that power distribution module 404 does not receive power from input power 548. At other times, power distribution module 404 may serve to charge battery 402 if necessary.

Other components of platform subsystem 502 may also include connector panel 320, control panel 322, and ring 324. Connector panel 320 may serve to connect different devices and components to system 300 and/or associated components and modules. Connector panel 320 may contain one or more ports that receive lines or connections from different components. For example, connector panel 320 may have a ground terminal port that may ground system 300 to other equipment, a port to connect foot pedal 544 to system 300, a port to connect to tracking subsystem 532, which may comprise position sensor 540, camera converter 542, and cameras 326 associated with camera stand 302. Connector panel 320 may also include other ports to allow USB, Ethernet, HDMI communications to other components, such as computer 408.

Control panel 322 may provide various buttons or indicators that control operation of system 300 and/or provide information regarding system 300. For example, control panel 322 may include buttons to power on or off system 300, lift or lower vertical column 312, and lift or lower stabilizers 520-526 that may be designed to engage casters 314 to lock system 300 from physically moving. Other buttons may stop system 300 in the event of an emergency, which may remove all motor power and apply mechanical brakes to stop all motion from occurring. Control panel 322 may also have indicators notifying the user of certain system conditions such as a line power indicator or status of charge for battery 402.

Ring 324 may be a visual indicator to notify the user of system 300 of different modes that system 300 is operating under and certain warnings to the user.

Computer subsystem 504 includes computer 408, display 304, and speaker 536. Computer 504 includes an operating system and software to operate system 300. Computer 504 may receive and process information from other components (for example, tracking subsystem 532, platform subsystem 502, and/or motion control subsystem 506) in order to display information to the user. Further, computer subsystem 504 may also include speaker 536 to provide audio to the user.

Tracking subsystem 532 may include position sensor 504 and converter 542. Tracking subsystem 532 may correspond to camera stand 302 including camera 326 as described with respect to FIG. 3. Position sensor 504 may be camera 326. Tracking subsystem may track the location of certain markers that are located on the different components of system 300 and/or instruments used by a user during a surgical procedure. This tracking may be conducted in a manner consistent with the present disclosure including the use of infrared technology that tracks the location of active or passive elements, such as LEDs or reflective markers, respectively. The location, orientation, and position of structures having these types of markers may be provided to computer 408 which may be shown to a user on display 304. For example, a surgical instrument 608 having these types of markers and tracked in this manner (which may be referred to as a navigational space) may be shown to a user in relation to a three dimensional image of a patient's anatomical structure. Motion control subsystem 506 may be configured to physically move vertical column 312, upper arm 306, lower arm 308, or rotate end effector 310. The physical movement may be conducted through the use of one or more motors 510-518. For example, motor 510 may be configured to vertically lift or lower vertical column 312. Motor 512 may be configured to laterally move upper arm 308 around a point of engagement with vertical column 312 as shown in FIG. 3. Motor 514 may be configured to laterally move lower arm 308 around a point of engagement with upper arm 308 as shown in FIG. 3. Motors 516 and 518 may be configured to move end effector 310 in a manner such that one may control the roll and one may control the tilt, thereby providing multiple angles that end effector 310 may be moved. These movements may be achieved by controller 538 which may control these movements through load cells disposed on end effector 310 and activated by a user engaging these load cells to move system 300 in a desired manner.

Moreover, system 300 may provide for automatic movement of vertical column 312, upper arm 306, and lower arm 308 through a user indicating on display 304 (which may be a touchscreen input device) the location of a surgical instrument or component on three dimensional image of the patient's anatomy on display 304. The user may initiate this automatic movement by stepping on foot pedal 544 or some other input means.

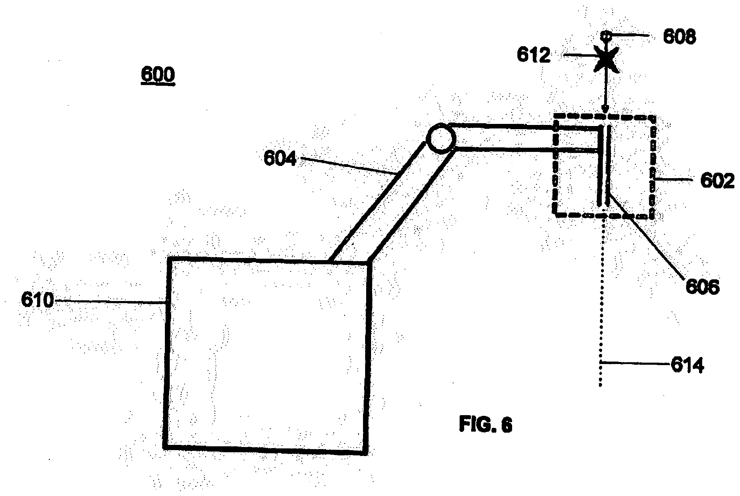

FIG. 6 illustrates a surgical robot system 600 consistent with an exemplary embodiment. Surgical robot system 600 may comprise end effector 602, robot arm 604, guide tube 606, instrument 608, and robot base 610. Instrument tool 608 may be attached to a tracking array 612 including one or more tracking markers and have an associated trajectory 614. Trajectory 614 may represent a path of movement that instrument tool 608 is configured to travel once it is positioned through or secured in guide tube 606, for example, a path of insertion of instrument tool 608 into a patient. In an exemplary operation, robot base 610 may be configured to be in electronic communication with robot arm 604 and end effector 602 so that surgical robot system 600 may assist a user (for example, a surgeon) in operating on the patient 210. Surgical robot system 600 may be consistent with previously described surgical robot system 100 and 300.

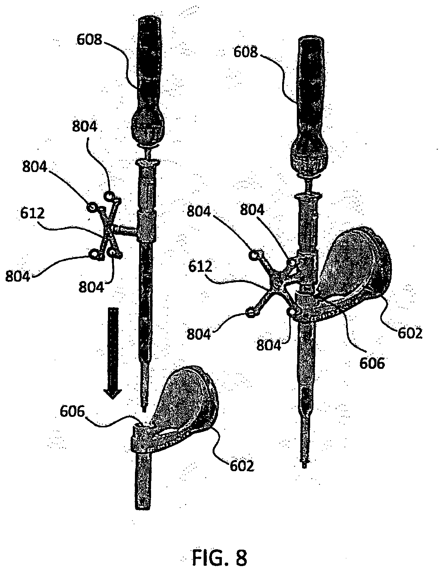

A tracking array 612 may be mounted on instrument 608 to monitor the location and orientation of instrument tool 608. The tracking array 612 may be attached to an instrument 608 and may comprise tracking markers 804. As best seen in FIG. 8, tracking markers 804 may be, for example, light emitting diodes and/or other types of reflective markers (e.g., markers 118 as described elsewhere herein). The tracking devices may be one or more line of sight devices associated with the surgical robot system. As an example, the tracking devices may be one or more cameras 200, 326 associated with the surgical robot system 100, 300 and may also track tracking array 612 for a defined domain or relative orientations of the instrument 608 in relation to the robot arm 604, the robot base 610, end effector 602, and/or the patient 210. The tracking devices may be consistent with those structures described in connection with camera stand 302 and tracking subsystem 532.

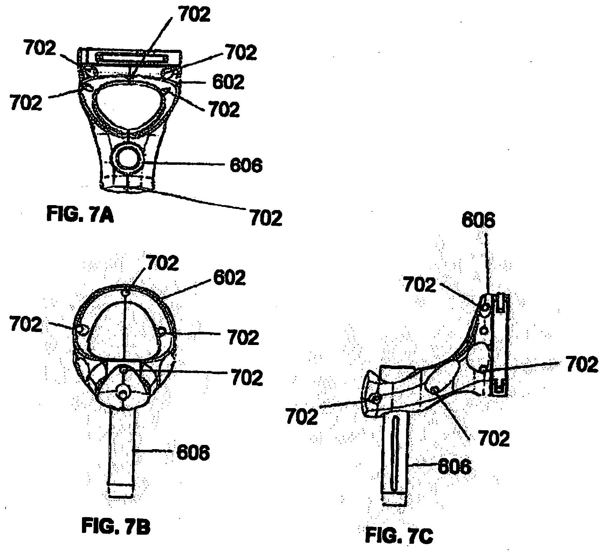

FIGS. 7A, 7B, and 7C illustrate a top view, front view, and side view, respectively, of end effector 602 consistent with an exemplary embodiment. End effector 602 may comprise one or more tracking markers 702. Tracking markers 702 may be light emitting diodes or other types of active and passive markers, such as tracking markers that have been previously described. In an exemplary embodiment, the tracking markers 702 are active infrared-emitting markers that are activated by an electrical signal (e.g., infrared light emitting diodes (LEDs)). Thus, tracking markers 702 may be activated such that the infrared markers 702 are visible to the camera 200, 326 or may be deactivated such that the infrared markers 702 are not visible to the camera 200, 326. Thus, when the markers 702 are active, the end effector 602 may be controlled by the system 100, 300, 600, and when the markers 702 are deactivated, the end effector 602 may be locked in position and unable to be moved by the system 100, 300, 600.

Markers 702 may be disposed on or within end effector 602 in a manner such that the markers 702 are visible by one or more cameras 200, 326 or other tracking devices associated with the surgical robot system 100, 300, 600. The camera 200, 326 or other tracking devices may track end effector 602 as it moves to different positions and viewing angles by following the movement of tracking markers 702. The location of markers 702 and/or end effector 602 may be shown on a display 110, 304 associated with the surgical robot system 100, 300, 600, for example, display 110 as shown in FIG. 2 and/or display 304 shown in FIG. 3. This display 110, 304 may allow a user to ensure that end effector 602 is in a desirable position in relation to robot arm 604, robot base 610, the patient 210, and/or the user.

For example, as shown in FIG. 7A, markers 702 may be placed around the surface of end effector 602 so that a tracking device placed away from the surgical field 208 and facing toward the robot 102, 301 and the camera 200, 326 is able to view at least 3 of the markers 702 through a range of common orientations of the end effector 602 relative to the tracking device 100, 300, 600. For example, distribution of markers 702 in this way allows end effector 602 to be monitored by the tracking devices when end effector 602 is translated and rotated in the surgical field 208.

In addition, in exemplary embodiments, end effector 602 may be equipped with infrared (IR) receivers that can detect when an external camera 200, 326 is getting ready to read markers 702. Upon this detection, end effector 602 may then illuminate markers 702. The detection by the IR receivers that the external camera200, 326 is ready to read markers 702 may signal the need to synchronize a duty cycle of markers 702, which may be light emitting diodes, to an external camera200, 326. This may also allow for lower power consumption by the robotic system as a whole, whereby markers 702 would only be illuminated at the appropriate time instead of being illuminated continuously. Further, in exemplary embodiments, markers 702 may be powered off to prevent interference with other navigation tools, such as different types of surgical instruments 608.

FIG. 8 depicts one type of surgical instrument 608 including a tracking array 612 and tracking markers 804. Tracking markers 804 may be of any type described herein including but not limited to light emitting diodes or reflective spheres. Markers 804 are monitored by tracking devices associated with the surgical robot system 100, 300, 600 and may be one or more of the line of sight cameras 200, 326. The cameras 200, 326 may track the location of instrument 608 based on the position and orientation of tracking array 612 and markers 804. A user, such as a surgeon 120, may orient instrument 608 in a manner so that tracking array 612 and markers 804 are sufficiently recognized by the tracking device or camera 200, 326 to display instrument 608 and markers 804 on, for example, display 110 of the exemplary surgical robot system.

The manner in which a surgeon 120 may place instrument 608 into guide tube 606 of the end effector 602 and adjust the instrument 608 is evident in FIG. 8. The hollow tube or guide tube 114, 606 of the end effector 112, 310, 602 is sized and configured to receive at least a portion of the surgical instrument 608. The guide tube 114, 606 is configured to be oriented by the robot arm 104 such that insertion and trajectory for the surgical instrument 608 is able to reach a desired anatomical target within or upon the body of the patient 210. The surgical instrument 608 may include at least a portion of a generally cylindrical instrument. Although a screw driver is exemplified as the surgical tool 608, it will be appreciated that any suitable surgical tool 608 may be positioned by the end effector 602. By way of example, the surgical instrument 608 may include one or more of a guide wire, cannula, a retractor, a drill, a reamer, a screw driver, an insertion tool, a removal tool, or the like. Although the hollow tube 114, 606 is generally shown as having a cylindrical configuration, it will be appreciated by those of skill in the art that the guide tube 114, 606 may have any suitable shape, size and configuration desired to accommodate the surgical instrument 608 and access the surgical site.

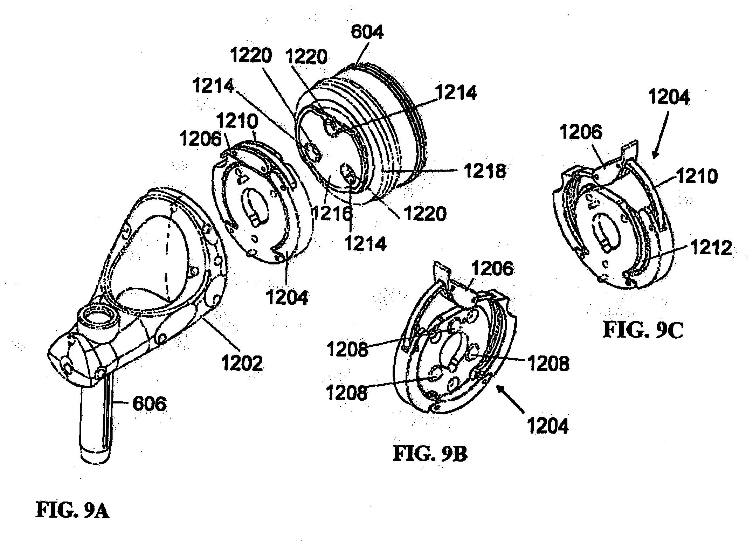

FIGS. 9A-9C illustrate end effector 602 and a portion of robot arm 604 consistent with an exemplary embodiment. End effector 602 may further comprise body 1202 and clamp 1204. Clamp 1204 may comprise handle 1206, balls 1208, spring 1210, and lip 1212. Robot arm 604 may further comprise depressions 1214, mounting plate 1216, lip 1218, and magnets 1220.

End effector 602 may mechanically interface and/or engage with the surgical robot system and robot arm 604 through one or more couplings. For example, end effector 602 may engage with robot arm 604 through a locating coupling and/or a reinforcing coupling. Through these couplings, end effector 602 may fasten with robot arm 604 outside a flexible and sterile barrier. In an exemplary embodiment, the locating coupling may be a magnetically kinematic mount and the reinforcing coupling may be a five bar over center clamping linkage.

With respect to the locating coupling, robot arm 604 may comprise mounting plate 1216, which may be non-magnetic material, one or more depressions 1214, lip 1218, and magnets 1220. Magnet 1220 is mounted below each of depressions 1214. Portions of clamp 1204 may comprise magnetic material and be attracted by one or more magnets 1220. Through the magnetic attraction of clamp 1204 and robot arm 604, balls 1208 become seated into respective depressions 1214. For example, balls 1208 as shown in FIG. 9B would be seated in depressions 1214 as shown in FIG. 9A. This seating may be considered a magnetically-assisted kinematic coupling. Magnets 1220 may be configured to be strong enough to support the entire weight of end effector 602 regardless of the orientation of end effector 602. The locating coupling may be any style of kinematic mount that uniquely restrains six degrees of freedom.

With respect to the reinforcing coupling, portions of clamp 1204 may be configured to be a fixed ground link and as such clamp 1204 may serve as a five bar linkage. Closing clamp handle 1206 may fasten end effector 602 to robot arm 604 as lip 1212 and lip 1218 engage clamp 1204 in a manner to secure end effector 602 and robot arm 604. When clamp handle 1206 is closed, spring 1210 may be stretched or stressed while clamp 1204 is in a locked position. The locked position may be a position that provides for linkage past center. Because of a closed position that is past center, the linkage will not open absent a force applied to clamp handle 1206 to release clamp 1204. Thus, in a locked position end effector 602 may be robustly secured to robot arm 604.

Spring 1210 may be a curved beam in tension. Spring 1210 may be comprised of a material that exhibits high stiffness and high yield strain such as virgin PEEK (poly-ether-etherketone). The linkage between end effector 602 and robot arm 604 may provide for a sterile barrier between end effector 602 and robot arm 604 without impeding fastening of the two couplings.

The reinforcing coupling may be a linkage with multiple spring members. The reinforcing coupling may latch with a cam or friction based mechanism. The reinforcing coupling may also be a sufficiently powerful electromagnet that will support fastening end-effector 102 to robot arm 604. The reinforcing coupling may be a multi-piece collar completely separate from either end effector 602 and/or robot arm 604 that slips over an interface between end effector 602 and robot arm 604 and tightens with a screw mechanism, an over center linkage, or a cam mechanism.

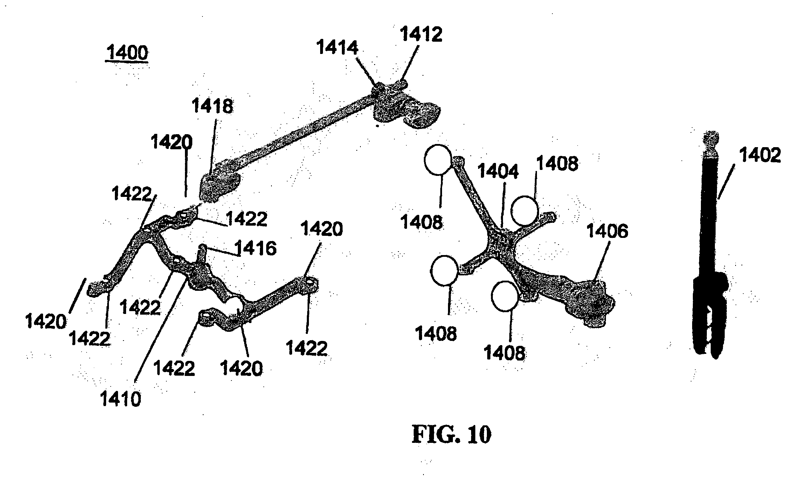

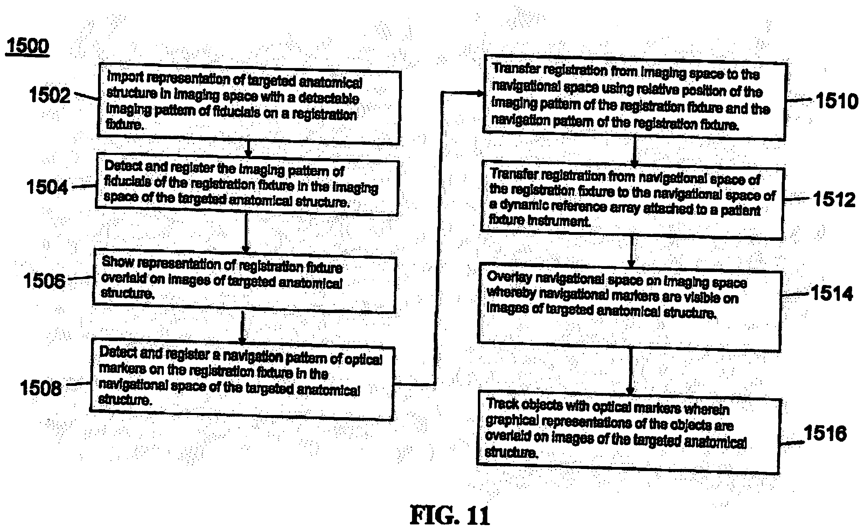

Referring to FIGS. 10 and 11, prior to or during a surgical procedure, certain registration procedures may be conducted in order to track objects and a target anatomical structure of the patient 210 both in a navigation space and an image space. In order to conduct such registration, a registration system 1400 may be used as illustrated in FIG. 10.

In order to track the position of the patient 210, a patient tracking device 116 may include a patient fixation instrument 1402 to be secured to a rigid anatomical structure of the patient 210 and a dynamic reference base (DRB) 1404 may be securely attached to the patient fixation instrument 1402. For example, patient fixation instrument 1402 may be inserted into opening 1406 of dynamic reference base 1404. Dynamic reference base 1404 may contain markers 1408 that are visible to tracking devices, such as tracking subsystem 532. These markers 1408 may be optical markers or reflective spheres, such as tracking markers, as previously discussed herein.

Patient fixation instrument 1402 is attached to a rigid anatomy of the patient 210 and may remain attached throughout the surgical procedure. In an exemplary embodiment, patient fixation instrument 1402 is attached to a rigid area of the patient 210, for example, a bone that is located away from the targeted anatomical structure subject to the surgical procedure. In order to track the targeted anatomical structure, dynamic reference base 1404 is associated with the targeted anatomical structure through the use of a registration fixture that is temporarily placed on or near the targeted anatomical structure in order to register the dynamic reference base 1404 with the location of the targeted anatomical structure.

A registration fixture 1410 is attached to patient fixation instrument 1402 through the use of a pivot arm 1412. Pivot arm 1412 is attached to patient fixation instrument 1402 by inserting patient fixation instrument 1402 through an opening 1414 of registration fixture 1410. Pivot arm 1412 is attached to registration fixture 1410 by, for example, inserting a knob 1416 through an opening 1418 of pivot arm 1412.

Using pivot arm 1412, registration fixture 1410 may be placed over the targeted anatomical structure and its location may be determined in an image space and navigation space using tracking markers 1420 and/or fiducials 1422 on registration fixture 1410. Registration fixture 1410 may contain a collection of markers 1420 that are visible in a navigational space (for example, markers 1420 may be detectable by tracking subsystem 532). Tracking markers 1420 may be optical markers visible in infrared light as previously described herein. Registration fixture 1410 may also contain a collection of fiducials 1422, for example, such as bearing balls, that are visible in an imaging space (for example, a three dimension CT image). As described in greater detail with respect to FIG. 11, using registration fixture 1410, the targeted anatomical structure may be associated with dynamic reference base 1404 thereby allowing depictions of objects in the navigational space to be overlaid on images of the anatomical structure. Dynamic reference base 1404, located at a position away from the targeted anatomical structure, may become a reference point thereby allowing removal of registration fixture 1410 and/or pivot arm 1412 from the surgical area.

FIG. 11 provides an exemplary method 1500 for registration consistent with the present disclosure. Method 1500 begins at step 1502 wherein a graphical representation (or image(s)) of the targeted anatomical structure may be imported into system 100, 300 600, for example computer 408. The graphical representation may be three dimensional CT or a fluoroscope scan of the targeted anatomical structure of the patient 210 which includes registration fixture 1410 and a detectable imaging pattern of fiducials 1420.

At step 1504, an imaging pattern of fiducials 1420 is detected and registered in the imaging space and stored in computer 408. Optionally, at this time at step 1506, a graphical representation of the registration fixture 1410 may be overlaid on the images of the targeted anatomical structure.

At step 1508, a navigational pattern of registration fixture 1410 is detected and registered by recognizing markers 1420. Markers 1420 may be optical markers that are recognized in the navigation space through infrared light by tracking subsystem 532 via position sensor 540. Thus, the location, orientation, and other information of the targeted anatomical structure is registered in the navigation space. Therefore, registration fixture 1410 may be recognized in both the image space through the use of fiducials 1422 and the navigation space through the use of markers 1420. At step 1510, the registration of registration fixture 1410 in the image space is transferred to the navigation space. This transferal is done, for example, by using the relative position of the imaging pattern of fiducials 1422 compared to the position of the navigation pattern of markers 1420.

At step 1512, registration of the navigation space of registration fixture 1410 (having been registered with the image space) is further transferred to the navigation space of dynamic registration array 1404 attached to patient fixture instrument 1402. Thus, registration fixture 1410 may be removed and dynamic reference base 1404 may be used to track the targeted anatomical structure in both the navigation and image space because the navigation space is associated with the image space.

At steps 1514 and 1516, the navigation space may be overlaid on the image space and objects with markers visible in the navigation space (for example, surgical instruments 608 with optical markers 804). The objects may be tracked through graphical representations of the surgical instrument 608 on the images of the targeted anatomical structure.

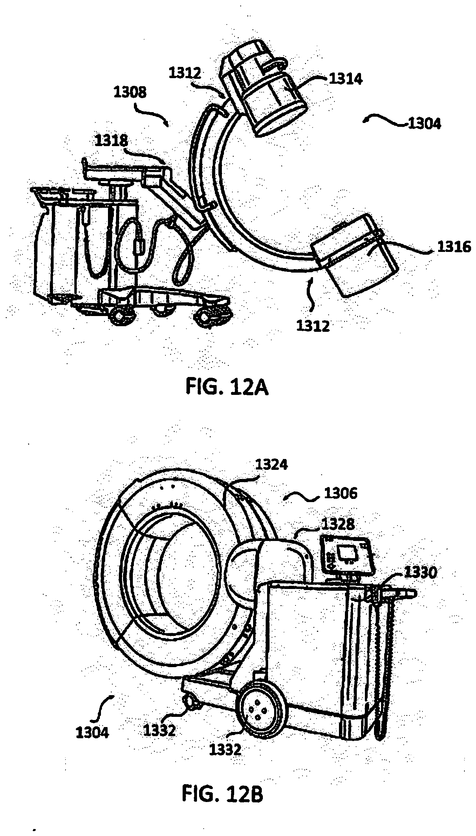

FIGS. 12A-12B illustrate imaging devices 1304 that may be used in conjunction with robot systems 100, 300, 600 to acquire pre-operative, intra-operative, post-operative, and/or real- time image data of patient 210. Any appropriate subject matter may be imaged for any appropriate procedure using the imaging system 1304. The imaging system 1304 may be any imaging device such as imaging device 1306 and/or a C-arm 1308 device. It may be desirable to take x-rays of patient 210 from a number of different positions, without the need for frequent manual repositioning of patient 210 which may be required in an x-ray system. As illustrated in Figure 12A, the imaging system 1304 may be in the form of a C-arm 1308 that includes an elongated C-shaped member terminating in opposing distal ends 1312 of the "C" shape. C-shaped member 1130 may further comprise an x-ray source 1314 and an image receptor 1316. The space within C-arm 1308 of the arm may provide room for the physician to attend to the patient substantially free of interference from x-ray support structure 1318. As illustrated in FIG. 12B, the imaging system may include imaging device 1306 having a gantry housing 1324 attached to a support structure imaging device support structure 1328, such as a wheeled mobile cart 1330 with wheels 1332, which may enclose an image capturing portion, not illustrated. The image capturing portion may include an x-ray source and/or emission portion and an x-ray receiving and/or image receiving portion, which may be disposed about one hundred and eighty degrees from each other and mounted on a rotor (not illustrated) relative to a track of the image capturing portion. The image capturing portion may be operable to rotate three hundred and sixty degrees during image acquisition. The image capturing portion may rotate around a central point and/or axis, allowing image data of patient 210 to be acquired from multiple directions or in multiple planes. Although certain imaging systems 1304 are exemplified herein, it will be appreciated that any suitable imaging system may be selected by one of ordinary skill in the art.

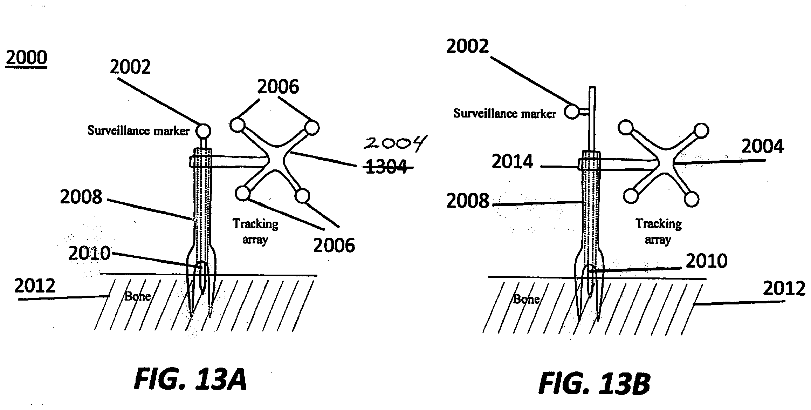

Referring now to FIGs. 7. 13A,13B of the present disclosure, exemplary embodiments of a surveillance marker consistent with the present invention are illustrated. FIGs. 13A-13B depicts a system 2000 including a surveillance maker 2002, a dynamic reference base (DRB) 2004, which may be a tracking array having array markers 2006, a DRB post 2008, and a surveillance marker post 2010. Also depicted is a patient's bone 2012. In this configuration, surveillance marker 2002 is on surveillance marker post 2010 that is within the hollow center or channel of the main shaft of the DRB post 2008. Surveillance marker post 2010 could consist of hard metal with a sharp, smooth tip for driving into bone with a mallet, or an end-threaded tip for drilling into bone. If DRB 2004 is bumped or dislodged, the tracking array with array markers 2006 would shift relative to the position of surveillance marker 2002 despite the close proximity of the spikes holding DRB 2004 to bone 2012 and the tip of the surveillance post 2010 for the surveillance marker 2002. Post 2010 to which surveillance marker 2002 is mounted is within the hollow main shaft of a spike or clamp to which DRB 2004 is mounted. There may be a loose tolerance between the wall of the hollow main shaft of DRB post 2008 and surveillance marker post 2010.

In the exemplary embodiment of FIG. 13A, with surveillance marker post 2010 encompassed by DRB post 2008, dislodgment and bending movement of DRB 2004 may cause DRB 2004 to press against surveillance marker post 2010 and cause it to move as well. However, since the attachment or entry point to bone 2012 is different for DRB post 2008 and surveillance marker post 2010, the axis of rotation of surveillance marker post 2010 and DRB 2004 may differ, meaning there may be a detectable change in position of surveillance marker 2002 relative to DRB 2004 even if the two structures touch. The amount of relative shift in position of surveillance marker 2002 and tracking markers 2006 if DRB 2004 is bumped may be greatest if surveillance marker post 2010 does not touch the inside wall of the hollow shaft of DRB post 2008. It may be beneficial to have a loose tolerance between surveillance marker post 2010 and inside wall of the DRB post 2010 for a more easily detectable effect. It may also be beneficial to at least begin surveillance with a condition where surveillance marker post 2010 is not touching the hollow wall of DRB post 2004, even if it will eventually touch during bending. To help ensure that surveillance marker post 2010 is not touching the hollow wall of DRB post 2008 during insertion, it may be beneficial to use a temporary centering guide such as a doughnut-shaped piece, through which surveillance marker post 2008 is inserted and which forces the surveillance marker post to the midline of the hollow shaft. After the post is inserted, the guide could be removed so that there remains loose tolerance between the hollow wall of the DRB post 2008 and surveillance marker post 2010. Such a guide could be used at the top of the DRB's tube region, at the bottom of the tube region, or both.

FIG. 13B is another configuration of system 2000 with surveillance marker 2002 offset from the midline of DRB post 2008. This configuration may avoid inadvertent rotation of the clamped DRB 2004 about the axis of the shaft when bumped. Such rotation may occur if clamp 2014 holding DRB 2004 in place is not sufficiently tightened and DRB 2004 is disturbed, even slightly. In the exemplary embodiment of Figure 13A, if rotation of DRB 2004 about DRB post 2008 occurred without any travel of DRB 2004 longitudinally along DRB post 2008, there may be minimal or undetectable relative movement of surveillance marker 2002 during rotational dislodgement since the position of surveillance marker 2002 is on or close to the axis of rotation of DRB 2004 rotational movement. In this event, surveillance marker 2002 may be offset from the midline or longitudinal axis of DRB post 2008 (for example by 1 cm or more), as shown in FIG. 13B. In this configuration, even a slight rotation of DRB 2004 about its mounting shaft would be detectable relative to the unmoved surveillance marker 2002, since surveillance marker 2002 is not on the axis of rotation of DRB 2004. This configuration may also give a distal region on surveillance marker post 2010 that can serve as the head to be struck with a hammer for driving it into bone, or a region to clamp in the chuck of a drill if it is to be drilled into bone. If surveillance marker 2002 is attached centrally to post 2010, a cap or other feature may be added to allow it to be inserted without damaging the surface of surveillance marker 2002, which may be coated with reflective paint. Additionally, during insertion, this configuration may allow surveillance marker 2002 to be manually rotated and positioned pointing toward the tracking cameras for better visibility.

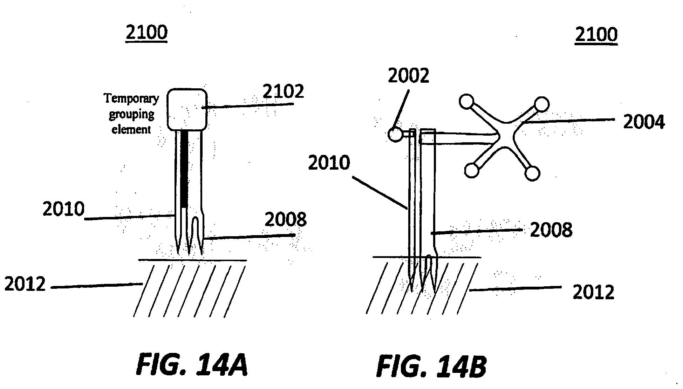

FIGs. 14A-B illustrates an example of a system 2100, not forming part of the claimed invention, that includes some components as previously described. System 2100 also includes a temporary grouping element 2102. In system 2100, surveillance marker 2002 may be attached through the same incision into a patient, but not physically connected to DRB 2004 or DRB post 2008. One manner to do this may be to use temporary grouping element 2102 to hold surveillance post 2010 and DRB post 2008 so that these components may be inserted as a unit. These components may then become independent after temporary grouping element 2102 is removed as shown in FIG. 14B.

In FIG. 14A, DRB post 2008 and surveillance marker post 2010 may be inserted into bone 2012 simultaneously while being held together at a desired spacing with temporary grouping element 2102. After DRB post 2008 and surveillance marker post 2010 have been inserted, temporary grouping element 2102 may be removed, and DRB 2004 and surveillance marker 2002 may be attached, which may be anchored in independent pieces of bone. Temporary grouping element 2102 may also be an impaction cap to be struck by a hammer during insertion.

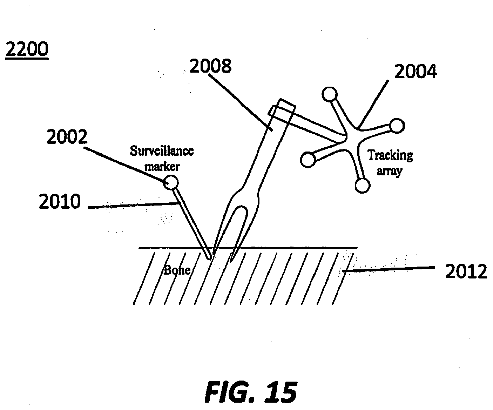

FIG. 15 illustrates an example 2200 which does not form part of the claimed invention. In this configuration it may be possible to attach surveillance marker 2002 through the same incision of the patient but not physically connected to DRB 2004. In FIG. 15, different insertion angles may be used, with trajectories of the surveillance marker post 2010 and DRB post 2008 within the same incision (not shown).

The attachment of surveillance marker 2002 as described with regard to FIGs. 13-15 may allow application of a surveillance marker, which has demonstrated benefits, through a single incision instead of requiring multiple incisions which may result in less time in surgery and less discomfort for the patient. While the invention has been disclosed in connection with the preferred embodiments shown and described in detail, various modifications and improvements thereon will become readily apparent to those skilled in the art. Accordingly, the scope of the present invention is not to be limited by the foregoing examples, but is to be understood in the broadest sense allowable by law.

## Claims

1. A system for use in monitoring registration of a patient to a surgical robot, said system comprising:
a computer (408);
a tracking camera (200) electronically coupled to the computer (408);
a surgical robot (102) electronically coupled to the computer;
a dynamic reference base (2004), including at least one array marker (2006) configured to be viewed by the tracking camera;
a dynamic reference base post (2008) connected to the dynamic reference base (2004); and
a surveillance marker (2002) configured to be viewed by the tracking camera, the surveillance marker (2002) further configured to be disposed at a predetermined distance from the dynamic reference base (2004), wherein the surveillance marker (2002) is configured to be secured to the patient independently from the dynamic reference base (2004);
a surveillance marker post (2010), wherein the surveillance marker (2002) is configured to be disposed on the surveillance marker post (2010), **characterised in that** the surveillance marker post (2010) is configured to be disposed in a hollow channel of the dynamic reference base post (2008).

2. The system of claim 1, wherein the surveillance marker (2002) is configured to be registered to the dynamic reference base (2004).

3. The system of claim 2, wherein the system is configured to determine that the dynamic reference base (2004) has moved based on a change in the predetermined distance.

4. The system of claim 1, wherein the surveillance marker (2002) is configured to be off-set from a longitudinal axis of the dynamic reference base post (2008).

5. The system of claim 1, wherein the surveillance marker (2002) arid the at least one array marker (2006) are each an optical marker.

6. The system of claim 1, wherein the surveillance marker post (2010) and the dynamic reference base post (2008) are configured to be inserted into a single incision.

7. The system of claim 1, wherein the surveillance marker post (2010) is made of metal and contains a sharp tip configured to be driven into a bony structure.

8. The system of claim 1, wherein the surveillance marker post (2010) is configured to be disposed in a bony structure.

9. The system of claim 8, wherein the surveillance marker post (2010) and the dynamic reference base post (2008) are configured to be inserted into a single incision.

## Patentansprüche

1. System zur Anwendung beim Überwachen einer Registrierung eines Patienten an einem chirurgischen Roboter, wobei das System aufweist:
- einen Computer (408);
- eine Verfolgungskamera (200), die elektronisch mit dem Computer (408) verbunden ist;
- einen chirurgischen Roboter (102), der elektronisch mit dem Computer verbunden ist;
- eine dynamische Referenzbasis (2004), die zumindest einen Anordnungsmarker (2006) umfasst, der eingerichtet ist, um von der Verfolgungskamera betrachtet werden;
- einen dynamischen Referenzbasispfosten (2008), der mit der dynamischen Referenzbasis (2004) verbunden ist; und
- einen Überwachungsmarker (2002), der eingerichtet ist, um von der Verfolgungskamera betrachtet zu werden, wobei der Überwachungsmarker (2002) ferner eingerichtet ist, um in einem vorbestimmten Abstand von der dynamischen Referenzbasis (2004) angeordnet zu sein, wobei der Überwachungsmarker (2002) eingerichtet ist, um unabhängig von der dynamischen Referenzbasis (2004) am Patienten befestigt zu sein;
- einen Überwachungsmarkerpfosten (2010),
wobei der Überwachungsmarker (2002) eingerichtet ist, um auf dem Überwachungsmarkerpfosten (2010) angeordnet zu sein, **dadurch gekennzeichnet, dass**
- der Überwachungsmarkerpfosten (2010) eingerichtet ist, um in einem hohlen Kanal des dynamischen Referenzbasispfostens (2008) angeordnet zu sein.

2. System nach Anspruch 1, wobei der Überwachungsmarker (2002) eingerichtet ist, um bei der dynamischen Referenzbasis (2004) registriert zu werden.

3. System nach Anspruch 2, wobei das System eingerichtet ist, um zu bestimmen, dass sich die dynamische Referenzbasis (2004) auf der Basis einer Änderung der vorbestimmten Entfernung bewegt hat.

4. System nach Anspruch 1, wobei der Überwachungsmarker (2002) eingerichtet ist, um von einer Längsachse des dynamischen Referenzbasispfostens (2008) versetzt zu sein.

5. System nach Anspruch 1, wobei der Überwachungsmarker (2002) und der zumindest eine Anordnungsmarker (2006) jeweils optische Marker sind.

6. System nach Anspruch 1, wobei der Überwachungsmarkerpfosten (2010) und der dynamische Referenzbasispfosten (2008) eingerichtet sind, um in einen einzelnen Einschnitt eingesetzt zu werden.

7. System nach Anspruch 1, wobei der Überwachungsmarkerpfosten (2010) aus Metall hergestellt ist und eine scharfe Spitze enthält, die eingerichtet ist, um in eine knöcherne Struktur eingetrieben zu werden.

8. System nach Anspruch 1, wobei der Überwachungsmarkerpfosten (2010) eingerichtet ist, um in einer knöchernen Struktur angeordnet zu sein.

9. System nach Anspruch 8, wobei der Überwachungsmarkerpfosten (2010) und der dynamische Referenzbasispfosten (2008) eingerichtet sind, um in einen einzelnen Einschnitt eingesetzt zu werden.

## Revendications

1. Système destiné à être utilisé dans le suivi de la coïncidence d'un patient avec un robot chirurgical, ledit système comprenant :
un ordinateur (408) ;
une caméra de traçage (200) couplée électroniquement à l'ordinateur (408) ;
un robot chirurgical (102) couplé électroniquement à l'ordinateur ;
une base de référence dynamique (2004), incluant au moins un marqueur d'ensemble (2006) configuré pour être vu par la caméra de traçage ;
un support de base de référence dynamique (2008) relié à la base de référence dynamique (2004) ; et
un marqueur de surveillance (2002) configuré pour être vu par la caméra de traçage, le marqueur de surveillance (2002) configuré en outre pour être disposé à une distance prédéterminée de la base de référence dynamique (2004), où le marqueur de surveillance (2002) est configuré pour être fixé au patient indépendamment de la base de référence dynamique (2004) ;
un support de marqueur de surveillance (2010),
où le marqueur de surveillance (2002) est configuré pour être disposé sur le support de marqueur de surveillance (2010), **caractérisé en ce que** le support de marqueur de surveillance (2010) est configuré pour être disposé dans un canal creux du support de base de référence dynamique (2008).

2. Système selon la revendication 1, où le marqueur de surveillance (2002) est configuré pour être en coïncidence avec la base de référence dynamique (2004).

3. Système selon la revendication 2, où le système est configuré pour déterminer que la base de référence dynamique (2004) s'est déplacée sur la base d'un changement dans la distance prédéterminée.

4. Système selon la revendication 1, où le marqueur de surveillance (2002) est configuré pour être décalé d'un axe longitudinal du support de base de référence dynamique (2008).

5. Système selon la revendication 1, où le marqueur de surveillance (2002) et le au moins un marqueur d'ensemble (2006) sont chacun un marqueur optique.

6. Système selon la revendication 1, où le support de marqueur de surveillance (2010) et le support de base de référence dynamique (2008) sont configurés pour être insérés dans une seule incision.

7. Système selon la revendication 1, où le support de marqueur de surveillance (2010) est fait de métal et contient une pointe acérée configurée pour être engagée dans une structure osseuse.

8. Système selon la revendication 1, où le support de marqueur de surveillance (2010) est configuré pour être disposé dans une structure osseuse.

9. Système selon la revendication 8, où le support de marqueur de surveillance (2010) et le support de base de référence dynamique (2008) sont configurés pour être insérés dans une seule incision.
